# EUROPÄISCHE PATENTSCHRIFT

(11) **EP 3 503 820 B1**
(45) Veröffentlichungstag und Bekanntmachung des Hinweises auf die Patenterteilung: **29.04.2020**
(21) Anmeldenummer: 17791946.1
(22) Anmeldetag: 29.09.2017
(51) Int. Cl.: A61B 17/295, A61B 18/14, A61B 17/29, A61B 18/00

(54) **CHIRURGISCHES MAULINSTRUMENT UMFASSEND EIN WURFHEBELSYSTEM**
SURGICAL JAW-TYPE INSTRUMENT COMPRISING A COUNTERBALANCED LEVER SYSTEM
INSTRUMENT CHIRURGICAL À MÂCHOIRES COMPRENANT UN SYSTÈME DE LEVIER MONOBRAS

(30) Priorität: 29.09.2016 DE 102016118482
(43) Veröffentlichungstag der Anmeldung: 03.07.2019
(73) Patentinhaber: Olympus Winter & Ibe GmbH, 22045 Hamburg (DE)
(72) Erfinder: MIERSCH, Hannes, 22149 Hamburg (DE); RÜHS, Andreas, 22926 Ahrensburg (DE); VOGT, Simon, 23560 Lübeck (DE)
(74) Vertreter: Meissner Bolte Partnerschaft mbB
(86) Internationale Anmeldenummer: PCT/EP2017/074850
(87) Internationale Veröffentlichungsnummer: WO 2018/060464

(56) Entgegenhaltungen:
- EP-A1- 2 777 586
- EP-A1- 2 853 221
- WO-A1-2015/069719
- US-A1- 2012 022 527
- US-A1- 2014 135 758
- US-A1- 2016 199 084

## Beschreibung

Die Erfindung betrifft ein chirurgisches Maulinstrument der im Oberbegriff des Anspruches 1 genannten Art.

Medizinische Handinstrumente der gattungsgemäßen Art werden üblicherweise für laparoskopische Eingriffe im Bauchraum eines Patienten verwendet. Ein an einem Schaft getragener Endeffektor wird dabei durch eine Öffnung der Bauchdecke hindurch in die Bauchhöhle des Patienten geführt und kann dort zur Manipulation von Gewebe und Organen durch Bedienung eines proximal am Schaft getragenen Handgriffes gesteuert werden. Während der Operation ist es häufig erforderlich, Gewebe durch den Endeffektor durchtrennen zu lassen. Der Endeffektor kann zu diesem Zweck ein Maulteil umfassen, das in der Lage ist, Gewebe zwischen zwei Maulbranchen zu halten, und ferner eine Messeranordnung, die das zwischen den beiden Maulbranchen gehaltene Gewebe schneiden kann. Um eine ausreichende Anwendungsflexibilität während der Operation zu gewährleisten, ist es bei den genannten chirurgischen Schneideinstrumenten ferner wünschenswert, dass das Maulteil flexibel um die Achse des Instrumentenschaftes drehbar ist.

Solche chirurgischen Schneideinstrumente sind unter anderem in US 9,216,030 B2, EP 2 853 221 A1, US 2014/0135758 A1, EP 2 777 586 A1, US 2005/0107785 A1, US 2012/0022527 A1, WO 2015/069719 A1, US 2016/0199084 A1 und US 2004/0254573 A1 beschrieben und werden z.B. als Ligasure Impact (Covidien), Enseal G2 Super Jaw (Ethicon) und HALO Cutting Forceps (OSTA) verkauft. Bei diesen Schneidinstrumenten wird eine Schneidklinge über die gesamte Länge des Maulteils von proximal nach distal geführt. Die Schneidklinge wird bei den Instrumenten durch verschiedene griffseitige, mechanische Mechanismen entlang der Schaftachse bewegt. Die griffseitige Führungslänge des Bedienmechanismus' hängt von der Maullänge ab, über die die Schneidklinge transportiert werden soll.

Ferner weisen die Instrumente ein Drehrad auf, mit dessen Hilfe das Maulteil um die Schaftachse rotiert werden kann. Diese Rotation des Maulteils ist wünschenswert, um die Operation für den Chirurgen weiter zu vereinfachen. Das Drehrad kann in den Handgriff integriert und somit einhändig bedienbar sein oder am Schaft angebracht und somit nur zweihändig bedienbar sein. Es versteht sich, dass die einhändige Bedienbarkeit in diesem Falle deutlich zu bevorzugen ist, da der Chirurg auf diese Weise die andere Hand frei hat und mit ihr weitere Operationsinstrumente bedienen kann.

Die derzeit griffseitig verwendeten Mechanismen zur Führung der Schneidklinge über die gesamte Maullänge sind sehr komplex und aufwändig in der Herstellung oder sie benötigen viel Platz im Griff, so dass eine gleichzeitige Anbringung des Drehrads zur Maulrotation im Griff nicht mehr möglich ist. Dies betrifft insbesondere sehr lange Maulteile mit Längen von über 30 mm, z. B. Maulteile mit einer Länge von 40 mm. Andere Mechanismen verwenden platzsparender einen Zahnrad-basierten Mechanismus zum Antrieb der Schneidklinge. Diese sind jedoch sehr komplex und teuer in der Herstellung.

Aufgabe der vorliegenden Erfindung ist es daher, ein chirurgisches Maulinstrument mit einer Messeranordnung und einem um die Schaftachse drehbaren Maulteil zur Verfügung zu stellen, bei dem der Führungsmechanismus für die Schneidklinge platzsparend im Griff unterbringbar ist und niedrigere Produktionskosten aufweist. Es sollte durch die platzsparende Unterbringung im Griffteil sichergestellt sein, dass auch ein Drehrad für die Rotation des Maulteils um die Schaftachse an dem Griffteil angebracht werden kann, so dass eine einhändige Bedienung ermöglicht wird.

### Beschreibung der Erfindung

Gelöst wird diese Aufgabe durch ein chirurgisches Maulinstrument mit den Merkmalen von Anspruch 1. Insbesondere wird die Aufgabe durch ein chirurgisches Maulinstrument gelöst, das als Führungsmechanismus für die Schneidklinge einer Messeranordnung ein Wurfhebelsystem umfasst, d. h. ein System von Hebeln, das mindestens einen Wurfhebel umfasst.

In einem ersten Aspekt betrifft die Erfindung ein chirurgisches Maulinstrument, an dessen distalem Ende ein Maulteil angeordnet ist, das mit zwei Maulbranchen ausgebildet ist, wobei mindestens eine Maulbranche schwenkbar gelagert ist und am proximalen Ende des Maulinstruments ein Handgriff zur Betätigung des Maulteils angeordnet ist und das elektrochirurgische Maulinstrument eine reversibel in axialer Richtung verschiebbare Messeranordnung zum Schneiden von Gewebe zwischen den Maulbranchen und einen als Rohr ausgebildeten Schaft umfasst, an dessen distalem Ende das Maulteil und an dessen proximalen Ende der Handgriff angeordnet ist, wobei das Maulteil von einem den Schaft durchlaufenden Maulbetätigungselement betätigbar ist und wobei die Messeranordnung von einem den Schaft durchlaufenden Messerbetätigungselement betätigbar ist, und das Maulbetätigungselement von dem am proximalen Ende des Schaftes angeordneten Handgriff in Richtung des Schaftes längsverschiebbar ist, dadurch gekennzeichnet, dass das Messerbetätigungselement mittels eines Wurfhebelsystems in Richtung des Schaftes längsverschiebbar ist, wobei das Wurfhebelsystem ein Betätigungselement, ein Koppelglied und einen Wurfhebel umfasst und wobei das Messerbetätigungselement durch Betätigung des Betätigungselements innerhalb des Schaftes in Schaftrichtung längsverschiebbar ist.

Das erfindungsgemäße chirurgische Maulinstrument hat den Vorteil, dass das Wurfhebelsystem auf günstige Weise hergestellt werden und auf kleinem Raum in dem Handgriff untergebracht werden kann. Deshalb ist es möglich z. B. ein Drehelement zum Rotieren des Maulteils ebenfalls am Griff anzubringen und somit die einhändige Bedienung zu ermöglichen. Gleichzeitig kann durch die erfindungsgemäße Verwendung eines Wurfhebelsystems ein Hub der Messeranordnung nach distal von 40 mm oder mehr erreicht werden. So können auch sehr lange Maulteile einhändig bedient werden.

Das erfindungsgemäße chirurgische Maulinstrument kann z. B. zum mechanischen Schneiden von Körpergewebe verwendet werden. Darüber hinaus kann das Maulinstrument aber auch zum Manipulieren, z. B. zum Halten von Körpergewebe benutzt werden.

Das "distale" Ende (z. B. des Maulinstruments oder des Schaftes) ist jeweils dasjenige Ende, das sich während der bei Anwendung durch medizinisches Fachpersonal in der Regel am Weitesten von dem medizinischen Fachpersonal entfernt ist (d. h. dasjenige Ende, das während der Operation näher am Patienten ist). Dies entspricht der Standardbenennung auf dem Fachgebiet. Im Umkehrschluss ist das "proximale" Ende jeweils dasjenige Ende, das sich während der Anwendung durch medizinisches Fachpersonal in der Regel am Dichtesten am Fachpersonal befindet (d. h. dasjenige Ende, das während der Operation weiter vom Patienten entfernt ist).

Am distalen Ende des chirurgischen Maulinstruments ist ein Maulteil angeordnet, das mit zwei Maulbranchen ausgebildet ist. Das erfindungsgemäße Maulteil ist mit zwei Maulbranchen ausgebildet, wobei mindestens eine Maulbranche schwenkbar gelagert ist. Die schwenkbare Maulbranche ist gegen die zweite Maulbranche bewegbar und berührt diese im geschlossenen Zustand und/oder ist in der Lage, ein zu manipulierendes Objekt zwischen den Maulbranchen zu halten. In einer Ausführungsform ist das Maulteil mit zwei um eine Achse schwenkbar gelagerten Maulbranchen ausgebildet. Die Maulbranchen sind gegeneinander bewegbar und können sich gegenseitig und/oder ein zu manipulierendes Objekt im geschlossenen Zustand berühren. In einer Ausführungsform ist daran gedacht, dass das Maulteil als Zange bzw. Greifer mit zwei, zum Greifen und Halten geeigneten, beweglichen Maulbranchen ausgebildet ist.

Am proximalen Ende des erfindungsgemäßen Maulinstruments ist ferner ein Handgriff zur Betätigung des Maulteils angeordnet. Der Handgriff besteht vorzugsweise aus zwei Griffelementen, die jeweils einer Maulbranche zugeordnet sind. Alternativ kann eine erste Maulbranche dem Schaft des Maulinstruments und eine zweite Maulbranche einem Griffelement zugeordnet sein, z.B. durch Befestigung an dem mit dem Griffelement verbundenen Betätigungselement wie z.B. einer Betätigungsstange.

Das elektrochirurgische Maulinstrument umfasst eine reversibel in axialer Richtung verschiebbare Messeranordnung zum Schneiden von Gewebe zwischen den Maulbranchen. Die Messeranordnung kann im zurückgezogenen Zustand in dem Schaft des Maulinstruments verborgen sein.

Durch das Wurfhebelsystem des Maulinstruments kann die Messeranordnung reversibel in axialer Richtung verschoben werden. Das Wurfhebelsystem ist somit eine Einrichtung zur selektiven und reversiblen Aktivierung der in axialer Richtung verschiebbaren Messeranordnung. Bei dem Verschieben in axialer Richtung gleitet die Messeranordnung in der Regel durch Messerkanäle hindurch, sofern die Gewebekontaktflächen einander ausreichend angenähert sind. Ein Messerkanal kann in nur einer oder in beiden Maulbranchen auf bzw. in der Gewebekontaktfläche der Maulbranche ausgebildet sein.

Die Gewebekontaktfläche ist jeweils diejenige Fläche einer Maulbranche, die beim Halten von dem zu manipulierenden Gewebe zwischen den Maulbranchen, unmittelbar mit dem Gewebe in Kontakt ist. Mit anderen Worten ist die Gewebekontaktfläche diejenige Fläche der Maulbranche, die der zweiten Maulbranche zugewandt ist. Der Begriff "Fläche" ist hierbei nicht einschränkend auszulegen und bedeutet insbesondere nicht, dass die Gewebekontaktfläche eben ist. Stattdessen kann die Gewebekontaktfläche einer Maulbranche Strukturen, wie z. B. einen Messerkanal und/oder eine Riffelung aufweisen, welche die Grifffestigkeit verbessert.

Greifen die Maulbranchen Gewebe, das deutlich dicker ist als die Messeranordnung und der Durchmesser des Schafts oder des Maulteils, so dass die Maulbranchen einander nur wenig angenähert sind, so gleitet die Messeranordnung in der Regel aus dem Schaft heraus, ohne durch die Messerkanäle der Maulbranchen hindurchgeführt zu werden. Diese Situation kann in einer Ausführungsform, um ungewollte Schnittverletzungen zu vermeiden, durch einen Sicherheitsmechanismus verhindert werden, der sicherstellt, dass die Messeranordnung nur in axialer Richtung verschiebbar ist, sofern die Messeranordnung durch mindestens einen Messerkanal hindurch geführt wird. Solche Sicherheitsmechanismen sind Fachleuten bekannt und können zum Beispiel eine Befestigung der Messeranordnung an beiden oder einem Messerkanal umfassen.

Die Messeranordnung umfasst bevorzugt mindestens ein Messer mit mindestens einer Schneide in im Wesentlichen distaler Richtung, d. h. an ihrem distalen Ende. Die Schneidekante des Messers kann angeschrägt sein. Das Messer kann als Schneidblech ausgebildet sein.

Der Messerkanal einer Maulbranche sowie der komplementäre Messerkanal auf der anderen Maulbranche sind vorzugsweise Teile einer Messeranordnung. Die Messerkanäle erstrecken sich derart durch die Zentren der Maulbranchen, dass eine Klinge, die Teil der Messeranordnung ist, in axialer Richtung von proximal nach distal durch einen oder beide der Kanäle geführt werden und Gewebe schneiden kann, das zwischen den beiden Maulbranchen gehalten wird. Geeignete Messeranordnungen sind z. B. in US 2004/0254573 A1 und US 2005/0107785 A1 beschrieben.

In einer Ausführungsform ist das erfindungsgemäße chirurgische Maulinstrument dadurch gekennzeichnet, dass es einen als Rohr ausgebildeten Schaft umfasst, an dessen distalem Ende das Maulteil und an dessen proximalen Ende der Handgriff angeordnet ist, und dadurch gekennzeichnet, dass das Maulteil von einem den Schaft durchlaufenden Maulbetätigungselement betätigbar ist, welches von dem am proximalen Ende des Schaftes angeordneten Handgriff in Richtung des Schaftes längsverschiebbar ist. Mit dem langgestreckten Schaft sind solche Instrumente vor allem für laparoskopische Anwendungen im Bauchraum geeignet, bei denen ein relativ großer Abstand zwischen Operateur und Eingriffsstelle besteht. In einer Ausführungsform ist das Maulinstrument somit ein endoskopisches und/oder laparoskopisches Instrument. Mit einem kürzeren Schaft können solche Instrumente aber auch in der offenen Chirurgie verwendet werden. In einer alternativen Ausführungsform ist das Instrument daher ein Instrument, das für die offene Chirurgie geeignet ist.

Das Maulbetätigungselement ist vorzugsweise als Betätigungsrohr ausgeprägt. Dies bedeutet, dass das Maulbetätigungselement in Längsrichtung einen inneren Hohlraum aufweist, durch den z. B. das an anderer Stelle geschilderte Messerbetätigungselement hindurchgeführt werden kann.

Das Maulbetätigungselement ist von dem am proximalen Ende des Schaftes angeordneten Handgriff in Richtung des Schaftes längsverschiebbar. Die Längsverschiebung wird durch Betätigung des Handgriffs bewirkt. Durch die Längsverschiebung des Maulbetätigungselements wird das Maulteil geöffnet oder geschlossen, d. h. in Offenstellung oder in Geschlossenstellung bewegt.

Die Messeranordnung ist von einem den Schaft durchlaufenden Messerbetätigungselement betätigbar. Das Messerbetätigungselement ist vorzugsweise als Betätigungsstange ausgeprägt und kann z. B. im Inneren des Maulbetätigungselements, das in diesem Falle als Betätigungsrohr ausgebildet ist, verlaufen.

Das Messerbetätigungselement ist durch Betätigung des Betätigungselements innerhalb des Schaftes in Schaftrichtung längsverschiebbar. Die Längsverschiebung wird durch Betätigung des Betätigungselements des Wurfhebelsystems bewirkt. Durch die Längsverschiebung des Messerbetätigungselements wird die Messeranordnung in distaler bzw. proximaler Richtung verschoben. Ein Verschieben in distaler Richtung bewirkt einen Schnitt in Gewebe, das zwischen den Maulbranchen gehalten wird.

Das Messerbetätigungselement kann vorzugsweise um einen relativ großen Hub nach distal bzw. in Längsrichtung des Schaftes verschoben werden. Vorzugsweise ist das Messerbetätigungselement mittels des Betätigungselements um mindestens 30 mm in Längsrichtung des Schaftes verschiebbar, besonders bevorzugt um mindestens 40 mm. Ein derart großer Hub bei gleichzeitiger Anbringung eines Drehrades zur Rotation des Maulteils am Handgriff wird erfindungsgemäß durch die Verwendung eines Wurfhebelsystems erreicht. Das Messerbetätigungselement ist demnach z. B. um mindestens 30 mm, vorzugsweise um mindestens 40 mm, noch stärker bevorzugt um mindestens 50 mm, am stärksten bevorzugt um mindestens 60 mm in Längsrichtung des Schaftes verschiebbar. Das Messerbetätigungselement kann z. B. in einem Bereich von 0 mm bis 120 mm, vorzugsweise von 0 mm bis 90 mm, stärker vorzugsweise von 0 mm bis 60 mm, am stärksten bevorzugt von 0 mm bis 45 mm verschiebbar sein.

Da die Messeranordnung durch das Messerbetätigungselement in Längsrichtung des Schafts verschiebbar ist, ist die Messeranordnung ebenso weit verschiebbar wie das Messerbetätigungselement.

Das Messerbetätigungselement ist mittels eines Wurfhebelsystems in Richtung des Schaftes längsverschiebbar, wobei das Wurfhebelsystem ein Betätigungselement, ein Koppelglied und einen Wurfhebel umfasst. Das Wurfhebelsystem ist ein Koppelgetriebe. Wurfhebelsysteme bzw. Wurfhebelgetriebe sind Fachleuten bekannt und werden z. B. auf der Homepage der Digitalen Mechanismen- und Getriebebibliothek gezeigt (http://www.dmg-lib.org).

Das Betätigungselement, Koppelglied, Wurfhebel und Messerbetätigungselement sind derart bewegungsgekoppelt, dass die Bewegung des Betätigungselements mittels des Koppelglieds und des Wurfhebels das Messerbetätigungselement in Längsrichtung entlang des Schaftes bewegt. Zur Arretierung der Messeranordnung kann vorgesehen sein, dass das Betätigungselement in einer Arretierstellung verharrt und somit die Messeranordnung arretiert. Betätigungselement, Koppelglied und Wurfhebel sind vorzugsweise in einer gemeinsamen bzw. in parallelen Bewegungsebene(n) beweglich, weiter vorzugsweise ausschließlich in dieser gemeinsamen bzw. den parallelen Bewegungsebenen. Betätigungselement, Koppelglied und Wurfhebel sind vorzugsweise jeweils im Wesentlichen starre Elemente, weiter vorzugsweise jeweils starre, einteilige Elemente.

Das hierin erwähnte "Betätigungselement" ist ein Wurfhebelbetätigungselement. Die Betätigungsbewegung des zur Betätigung der Messeranordnung bzw. des Messerbetätigungselements vorgesehenen Betätigungselements verläuft vorzugsweise parallel zur Bewegungsebene der Griffstücke. Hierdurch kann das Betätigungselement mit einem Finger derselben Hand einfach bedient werden, die auch die Griffstücke bedient. Um die Bedienung mit einem Finger weiter zu erleichtern, kann das Betätigungselement eine räumlich an einen Finger angepasste, ergonomische Form und/oder Oberfläche aufweisen. So kann das Betätigungselement z. B. eine Krümmung aufweisen, in die der Finger des medizinischen Bedienungspersonals eingelegt werden kann, und/oder eine Riffelung der Oberfläche, die ein Verrutschen des Bedienfingers verhindert. Das Betätigungselement weist ferner eine längliche, vorzugsweise gekrümmt-längliche, Form auf. Das längliche Betätigungselement erstreckt sich vorzugsweise aus einem Griffteil des Maulinstruments in distaler, inferiorer oder distal-inferiorer Richtung heraus.

Wie hierin verwendet, bezeichnet "inferior" diejenige Richtung, die bei einer normalen Benutzungshaltung unterhalb des Schaftbereiches liegt. "Superior" ist diejenige Richtung, die bei einer normalen Benutzungshandlung oberhalb des Schaftbereiches liegt. Mit anderen Worten, weist die Achse, die sich von inferior nach superior durch die Bewegungsebene der Handgriffe erstreckt, einen 90° Winkel zur Längsachse des Schafts auf.

Das Betätigungselement ist bevorzugt ein Hebel zweiter oder dritter Klasse, bevorzugt dritter Klasse. Ein Hebel dritter Klasse ist, wie bekannt, ein Hebel dessen Auflagepunkt sich am einen Ende des Hebels befindet und bei dem sich ferner die Last, die zu überwinden ist, am weitesten vom Auflagepunkt entfernt befindet, während die Kraft zwischen Auflagepunkt und Last wirkt. Bei einem Hebel zweiter Klasse befindet sich der Auflagepunkt ebenfalls an einem Ende des Hebels, aber die Last, die zu überwinden ist, wirkt zwischen Auflagepunkt und Kraft.

Der Punkt des Betätigungselements, auf den die Last wirkt, wird durch die Verbindung zwischen dem Betätigungselement und dem Koppelglied gebildet. Das Betätigungselement ist in der Regel mittels eines Lagers, das eine rotatorische Bewegung zulässt, mit dem Koppelglied verbunden. Die rotatorische Bewegung ist vorzugsweise parallel zur Bewegungsebene der Griffstücke, ebenso wie die Bewegung des Betätigungselements, des Koppelglieds und des Wurfhebels. Die rotatorische Bewegung ist vorzugsweise parallel zur gemeinsamen Bewegungsebene von Betätigungselement, Koppelglied und Wurfhebel. Mit anderen Worten ist das Betätigungselement mit dem Koppelglied z. B. mittels eines Radiallagers verbunden. Zusätzlich ist es denkbar, dass das Lager weitere Freiheitsgrade aufweist, zum Beispiel eine translatorische Bewegung entlang der Längsachse des Betätigungselements oder des Koppelglieds.

In einer alternativen Ausführungsform kann die Verbindung zwischen dem Betätigungselement und dem Koppelglied auch starr ausgebildet sein. Weiter alternativ können Betätigungselement und Koppelglied als ein (starres) Teil ausgebildet sein.

Das Koppelglied verbindet das Betätigungselement (Wurfhebelbetätigungselement) mit dem Wurfhebel. Das Koppelglied weist eine längliche Form auf, optional eine längliche gekrümmte Form. Das Koppelglied erstreckt sich im Wesentlichen parallel zur Längsachse des Schaftes, wobei dies nicht ausschließt, dass die Längsachse des Koppelglieds gegenüber einer Parallelachse zur Schaftachse leicht (z. B. + / - 50°), z. B. in inferiore oder superiore Richtung, gekippt sein kann. Bevorzugt ist das Koppelglied mit seinem distaleren Ende (mittels des oben beschriebenen Lagers) mit dem Betätigungselement verbunden und mit seinem proximaleren Ende (mittels des an anderer Stelle hierin beschriebenen weiteren Lagers) mit dem Wurfhebel verbunden. Vorzugsweise ist das distalere Ende des Koppelglieds mit dem inferioreren Ende des Betätigungselements verbunden und das proximalere Ende des Koppelglieds mit dem inferioreren Ende des Wurfhebels.

Vorzugsweise ist das Koppelglied nur mit den anderen Komponenten des Wurfhebelsystems, d. h. dem Betätigungselement, dem Wurfhebel bzw. den entsprechenden Lagern und weiteren Lagerelementen verbunden. Dies bedeutet, dass das Koppelglied vorzugsweise nicht mit Teilen des Maulinstruments verbunden ist, die kein Teil des Wurfhebelsystems sind.

Der Punkt des Koppelglieds, auf den die Last wirkt, wird durch die Verbindung zwischen dem Koppelglied und dem Wurfhebel gebildet. Das Koppelglied ist mittels eines Lagers, das eine rotatorische Bewegung zulässt, mit dem Wurfhebel verbunden. Die rotatorische Bewegung ist vorzugsweise parallel zur Bewegungsebene der Griffstücke. Die rotatorische Bewegung ist vorzugsweise parallel zur gemeinsamen Bewegungsebene von Betätigungselement, Koppelglied und Wurfhebel. Mit anderen Worten ist das Koppelglied mit dem Wurfhebel z. B. mittels eines Radiallagers verbunden. Zusätzlich ist es denkbar, dass das Lager weitere Freiheitsgrade aufweist und zum Beispiel eine translatorische Bewegung entlang der Längsachse des Koppelglied oder des Wurfhebels erlaubt.

Der Wurfhebel verbindet das Koppelglied mit dem Messerbetätigungselement. Der Wurfhebel weist eine längliche Form auf, optional eine längliche gekrümmte Form. Das Koppelglied erstreckt sich vom proximaleren Ende des Koppelglieds vorzugsweise nach superior bzw. proximal-superior zum proximalen Ende des Messerbetätigungselements. Die Längsachse des Wurfhebels erstreckt sich somit vorzugsweise in superiorer oder proximal-superiorer Richtung (ausgehend vom proximaleren Ende des Koppelglieds).

Der Wurfhebel ist bevorzugt ein Hebel erster Klasse, dessen Auflagepunkt sich zwischen der wirkenden Kraft (durch das Koppelglied) und der Last (auf das Messerbetätigungselement) befindet. Bevorzugt befindet sich der Auflagepunkt im mittleren Abschnitt des Wurfhebels, besonders bevorzugt in der Mitte des Wurfhebels. Alternativ kann sich, je nach Bauform und benötigter Kraft und Hub, der Auflagepunkt des Wurfhebels auch an einer anderen Position befinden. Der Fachmann ist in der Lage, entsprechend der Erfordernisse eine geeignete Position für den Auflagepunkt des Wurfhebels zu bestimmen.

Der Auflagepunkt des Wurfhebels wird durch die Befestigung des Wurfhebels innerhalb des Griffteils gebildet. Der Auflagepunkt kann z. B. in einer Ausführungsform durch je einen Hohlzylinder in zwei Kunststoffhalbschalen gebildet werden, wobei die Hohlzylinder z. B. durch ein Spritzgussverfahren ausgebildet werden und die zwei Kunststoffschalen das Griffteil bilden. Die Enden der beiden Hohlzylinder liegen sich nach dem Zusammensetzen der Kunststoffhalbschalen gegenüber und könnten z. B. durch einen Stift, der in den Innenraum der Zylinder eingeführt wird, verbunden werden. Der Stift kann dann als drehbarer Lagerungsort für den Wurfhebel dienen. Der Stift kann zur Verbesserung der Stabilität aus einem metallischen Material sein oder einem Verbundmaterial sein, das ein metallisches Material umfasst.

Bevorzugt ist der Lastarm des Wurfhebels um eine senkrecht durch seinen Auflagepunkt und orthogonal zu der Längsachse des Schafts verlaufende Schwenkachse von -45° bis + 45° schwenkbar. Hierdurch werden extreme Minimalkräfte in den Start- und Endlagen vermieden. Die erzielbaren Schneidblechkräfte bei z. B. 30 N Fingerkraft liegen am Anfang bei etwa 14 N, steigen auf etwa 21 N an und fallen dann auf 10 N ab.

Die Anordnung der einzelnen Elemente des Wurfhebelsystems stellt sicher, dass die Elemente derart funktional miteinander verbunden sind, dass das Messerbetätigungselement durch Betätigung des Betätigungselements (Wurfhebelbetätigungselement) in Längsrichtung des Schafts bewegt werden kann (sowohl in distaler, als auch in proximaler Richtung). Somit ist das Betätigungselement mittels des Koppelglieds mit dem Wurfhebel (und auch dem Messerbetätigungselement) funktional verbunden.

Der Wurfhebel ist mit dem proximalen Endbereich des Messerbetätigungselements (funktional) verbunden. Insbesondere kann der Wurfhebel mittels eines Gleitlagers mit dem proximalen Endbereich des Messerbetätigungselements (funktional) verbunden sein.

Die Verbindung zwischen dem Messerbetätigungselement und dem Wurfhebel sollte bevorzugt nicht nur eine rotatorische Bewegung erlauben, sondern auch eine translatorische Bewegung des Messerbetätigungselements entlang der Längsachse des Wurfhebels. Auf diese Weise wird sichergestellt, dass das Messerbetätigungselement in einer geraden Bahn in oder parallel zur Längsachse des Schafts bewegt und nicht in eine Kreisbahn gezwungen wird. In einer bevorzugten Ausführungsform weist das Messerbetätigungselement somit an seinem proximalen Ende ein Wurfhebel-Verbindungselement auf, das einen Zapfenbereich mit rundem Querschnitt und einen Verdickungsbereich proximal des Zapfenbereichs umfasst, und der Wurfhebel weist an seinem mit dem Messerbetätigungselement verbundenen Ende einen Schlitz auf, wobei der Zapfenbereich durch den Schlitz derart hindurchgeführt ist, dass sich der Verdickungsbereich im Inneren des Wurfhebels befindet.

Der Wurfhebel kann somit wenigstens in dem Endbereich, der mit dem Messerbetätigungselement verbunden ist, wenigstens abschnittweise rohrförmig, d. h. in Form eines Hohlzylinders ausgebildet sein.

Das chirurgische Maulinstrument kann ferner ein Wurfhebelrückzugselement aufweisen, das den mit dem Messerbetätigungselement verbundenen Endbereich des Wurfhebels bei Loslassen des Betätigungselements durch das medizinische Personal automatisch in proximale Richtung bewegt. Alternativ kann das Wurfhebelrückzugselement auch mit dem Messerbetätigungselement verbunden sein und dieses (und damit auch den mit dem Messerbetätigungselement verbundenen Endbereich des Wurfhebels) in proximale Richtung bewegen. Das Wurfhebelrückzugselement kann z. B. eine Feder sein.

Die zuvor beschriebene Konstruktion erlaubt eine platzsparende Unterbringung des Wurfhebelsystems im Handgriff des Maulinstruments. Die platzsparende Unterbringung erlaubt darüber hinaus die Anbringung eines Drehelements zur Rotation des Maulteils um die Schaftachse an dem Handgriff und damit die einhändige Bedienung des Drehelements und der weiteren Funktionen des Handgriffs (u. a. des Wurfhebelsystems). In einer bevorzugten Ausführungsform ist das Maulteil somit mittels eines im proximalen Endbereich des chirurgischen Maulelements angeordneten Drehelements um die Längsachse des Schaftes drehbar. Vorzugsweise ist das Maulteil in einem Winkelbereich zwischen 0° und 360° um die Längsachse des Schaftes drehbar. Das Drehelement kann z. B. als Drehrad ausgebildet sein, z. B. als Drehrad, das an der superioren Seite des Maulinstruments ausgebildet ist.

In Übereinstimmung mit dem oben genannten Vorteilen der vorliegenden Erfindung, sind das Drehelement und das Betätigungselement derart am oder in der Nähe des Handgriffs angeordnet, dass eine einhändige Bedienung der Griffelemente des Handgriffs, des Drehelements und des Betätigungselements ermöglicht wird. Dies bedeutet, dass alle der vorgenannten Elemente mit einer Hand bedient werden können, vorzugsweise ohne dass das medizinische Bedienpersonal den Handgriff loslassen und/oder umgreifen muss.

### Kurze Beschreibung der Figuren

In den Zeichnungen sind Ausführungsbeispiele der Erfindung schematisch dargestellt. Es zeigen:
- **Fig. 1**: eine Seitenansicht eines erfindungsgemäßen Maulinstruments, wobei die Messeranordnung in distaler Richtung zwischen die Maulbranchen geschoben ist;
- **Fig. 2**: eine Seitenansicht eines erfindungsgemäßen Maulinstruments, wobei die Messeranordnung in proximale Richtung zurück verschoben und damit zum größten Teil im Schaft verborgen ist;
- **Fig. 3**: eine schematische Darstellung des Schwenkbereichs des Wurfhebels; und
- **Fig. 4**: eine schematische Darstellung einer Ausführungsform der Verbindung zwischen Wurfhebel und Messerbetätigungselement.

### Ausführungsbeispiele

Weitere Vorteile, Kennzeichen und Merkmale der vorliegenden Erfindung werden bei der nachfolgenden detaillierten Beschreibung von Ausführungsbeispielen anhand der beigefügten Zeichnungen deutlich. Allerdings ist die Erfindung nicht auf diese Ausführungsbeispiele beschränkt.

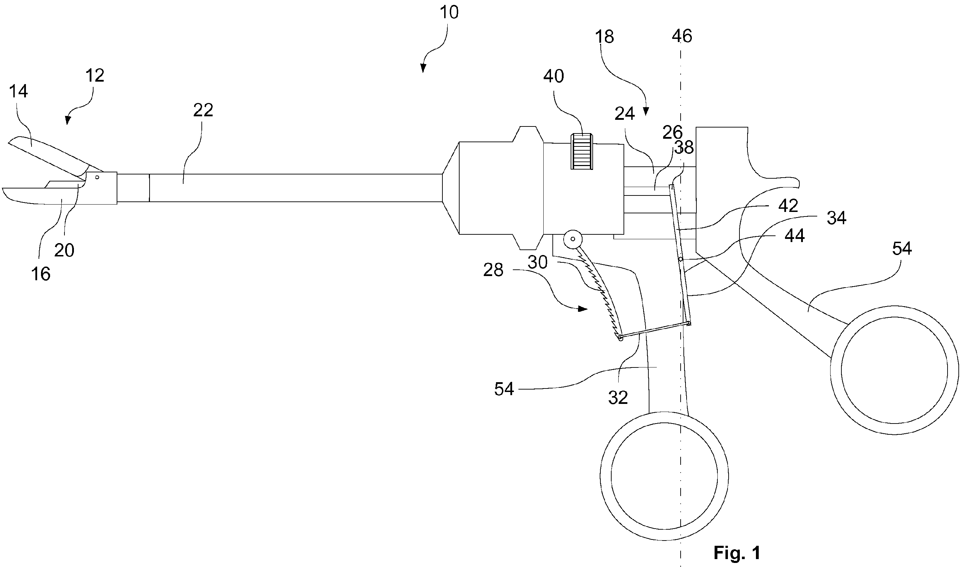

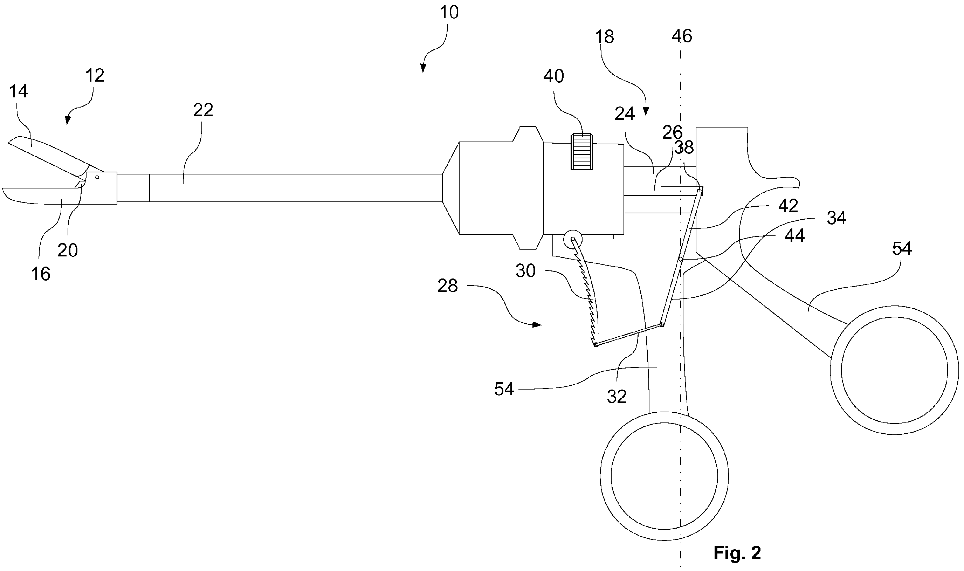

Fig. 1 und 2 zeigen Seitenansichten einer Ausführungsform des erfindungsgemäßen Maulinstruments 10, bei dem in Fig. 1 die Messeranordnung 20 in distale Richtung zwischen die beiden Maulbranchen 14, 16 geschoben ist und in Fig. 2 die Messeranordnung 20 in proximaler Richtung in den Schaft 22 zurückgezogen ist. Die Messeranordung 20 ist als Schneidblech ausgebildet. Die in Fig. 1 gezeigte Stellung zeigt den Prozess des Schneidens zwischen den beiden Maulbranchen 14, 16. Hier nicht dargestelltes Gewebe, das zwischen den beiden Maulbranchen 14, 16 gehalten wird, wird durch die Messeranordnung 20 durchtrennt. Die Maulbranchen 14, 16 können beide geschlossen sein. Es kann insbesondere vorgesehen sein, dass die Messeranordnung 20 nur in distaler Richtung zwischen die beiden Maulbranchen 14, 16 verschiebbar ist, wenn die Maulbranchen 14,16 geschlossen sind, d. h. deren Gewebekontaktflächen im Wesentlichen parallel zueinander stehen.

Das Maulinstrument 10 in Fig. 1 und 2 weist einen langgestreckten Schaft 22 auf und ist somit besonders für laparoskopische Operationen geeignet. Darüber hinaus weist das Maulinstrument 10 einen Handgriff 18 mit einem erfindungsgemäßen Wurfhebelsystem 28 auf.

Der Handgriff 18 weist zwei Griffelemente 54 auf, die unterschiedlich ausgebildet sein können. Es können beide oder nur eines der beiden Griffelemente 54 gegenüber dem Schaft 22 beweglich sein.

Ferner umfasst der Handgriff 18 ein Wurfhebelsystem 28, das zur Betätigung des Messerbetätigungselements 26 und somit der Messeranordnung 20 dient. Das Wurfhebelsystem 28 umfasst ein Betätigungselement 30, ein Koppelglied 32 und einen Wurfhebel 34. Das Betätigungselement 30 ist an dem Handgriff 18 befestigt. Die Verbindung zwischen Betätigungselement 30 und dem Handgriff 18 wird durch ein Lager bewirkt, das eine rotatorische Bewegung zulässt. Das dargestellte Betätigungselement 30 weist eine Riffelung seiner Oberfläche auf einer dem Finger des medizinischen Fachpersonals bei Betätigung des Betätigungselements zugewandten Seite auf. Hierdurch wird ein Verrutschen des Fingers verhindert.

An seinem inferioren Ende ist das Betätigungselement 30 mittels eines Lagers, das eine rotatorische Bewegung zulässt, mit dem Koppelglied 32 verbunden. Das Koppelglied 32 verbindet das Betätigungselement 30 mit dem Wurfhebel 34. Zu diesem Zweck ist auch die Verbindung zwischen dem proximaleren Ende des Koppelgliedes 32 und dem inferioren Ende des Wurfhebels 34 als ein Lager ausgestaltet, das eine rotatorische Bewegung zulässt. Weitere Freiheitsgrade sind für diese Verbindung denkbar.

Der Wurfhebel 34 weist einen Auflagepunkt 44 auf, der in der gezeigten Ausführungsform als Lager ausgebildet ist, das eine rotatorische Bewegung zulässt. Durch den Auflagepunkt 44 des Wurfhebels 34 verläuft die senkrecht durch den Auflagepunkt 44 und orthogonal zur Längsachse 23 des Schafts 22 verlaufende Schwenkachse 46. Der Lastarm 42 des Wurfhebels 34 ist um die Schwenkachse 46 von -45° bis + 45° schwenkbar.

Das superiore Ende des Wurfhebels 34 ist mit dem proximalen Ende des Messerbetätigungselements 26 mittels eines Wurfhebel-Verbindungselements 38 verbunden. Das dargestellte Wurfhebel-Verbindungselement 38 ist ein Lager, das eine rotatorische Bewegung und eine translatorische Bewegung zulässt. Alternativ kann das superiore Ende des Wurfhebels 34 mit dem proximalen Ende des Messerbetätigungselements 26 auch durch ein einfacheres Gleitlager 36 verbunden sein. Diese Ausführungsform ist nicht dargestellt.

Das Maulinstrument 10 in Fig. 1 und 2 umfasst ferner ein Drehelement 40, das superior von dem Betätigungselement 30 an dem Maulinstrument 10 angebracht ist. Das gezeigte Drehelement 40 ist als ein Drehrad ausgebildet und weist auf seiner Oberfläche eine Riffelung auf, um die Bedienung zu vereinfachen und insbesondere ein Abrutschen zu verhindern.

Fig. 1 zeigt ein bereits betätigtes Betätigungselement 30. Es ist erkennbar, dass das Betätigungselement 30, insbesondere dessen inferiores Ende, in proximale Richtung verschwenkt ist. Hierdurch wurde das Messerbetätigungselement 26 und in der Folge die Messeranordnung 20 in distaler Richtung zwischen die Maulbranchen 14,16 geschoben.

In Fig. 2 ist das Betätigungselement 30 relaxiert und in distaler Richtung verschoben. Hierdurch wurde das Messerbetätigungselement 26 und in der Folge die Messeranordnung 20 in proximale Richtung fast vollständig in den Schaft 22 zurückgezogen .

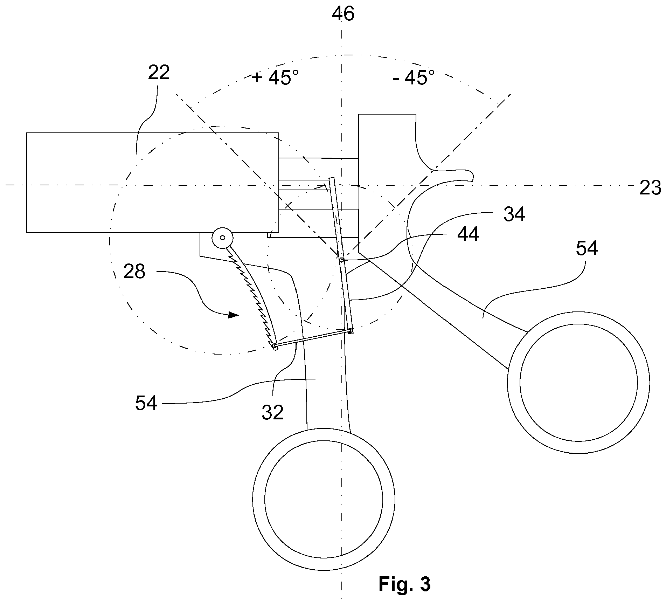

Fig. 3 zeigt eine detailliertere Darstellung der Schwenkbereiche des Betätigungselements 30 und insbesondere des Wurfhebels 34. Es ist erkennbar, dass das Betätigungselement 30 um seinen Befestigungspunkt am Handgriff 18 herum in einer rotatorischen Bewegung bewegt werden kann. Die rotatorische Bewegung des Betätigungselements 30 ist in der gezeigten Ausführungsform in einer Ebene parallel zur Bewegungsebene der Handgriffe 54.

Der Wurfhebel 34 kann ebenfalls um seinen Auflagepunkt 44 (Befestigungspunkt am Handgriff) herum in einer rotatorischen Bewegung bewegt werden. Die rotatorische Bewegung des Wurfhebels 34 ist in der gezeigten Ausführungsform in einer Ebene parallel zur Bewegungsebene der Handgriffe 54. Es ist erkennbar, dass der Lastarm 42 des Wurfhebels 28 um eine senkrecht durch seinen Auflagepunkt 44 und orthogonal zu der Längsachse 23 des Schafts 22 verlaufende Schwenkachse 46 von -45° bis + 45° schwenkbar ist.

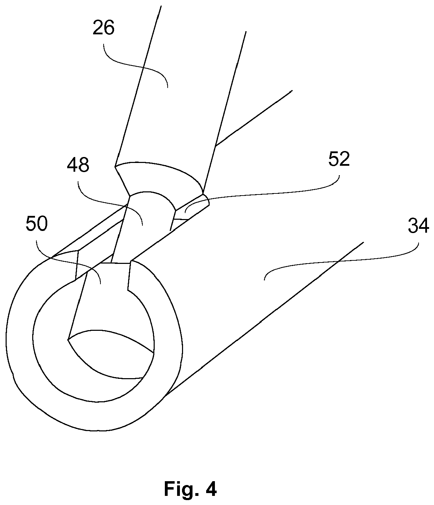

Fig. 4 zeigt eine schematische Darstellung einer Ausführungsform der Verbindung zwischen Wurfhebel 34 und Messerbetätigungselement 26. Das dargestellte Messerbetätigungselement 26 weist an seinem proximalen Ende ein Wurfhebel-Verbindungselement 38 auf, das einen Zapfenbereich 48 mit rundem Querschnitt und einen Verdickungsbereich 50 proximal des Zapfenbereichs 48 umfasst. Der Wurfhebel 34 weist an seinem mit dem Messerbetätigungselement 26 verbundenen Ende einen Schlitz 52 auf, wobei der Zapfenbereich 48 durch den Schlitz 52 derart hindurchgeführt ist, dass sich der Verdickungsbereich 50 im Inneren des Wurfhebels 34 befindet.

Obwohl die vorliegende Erfindung anhand der Ausführungsbeispiele detailliert beschrieben worden ist, ist für den Fachmann selbstverständlich, dass die Erfindung nicht auf diese Ausführungsbeispiele beschränkt ist, sondern dass vielmehr Abwandlungen in der Weise möglich sind, dass einzelne Merkmale weggelassen oder andersartige Kombinationen der vorgestellten Einzelmerkmale verwirklicht werden können, sofern der Schutzbereich der beigefügten Ansprüche nicht verlassen wird. Die vorliegende Offenbarung schließt sämtliche Kombinationen der vorgestellten Einzelmerkmale ein.

**Bezugszeichenliste**

| | | | |
|---|---|---|---|
| 10 | chirurgisches Maulinstrument | 48 | Zapfenbereich |
| 12 | Maulteil | 50 | Verdickungsbereich |
| 14 | Maulbranche | 52 | Schlitz |
| 16 | Maulbranche | 54 | Griffelement |
| 18 | Handgriff | | |
| 20 | Messeranordnung | | |
| 22 | Schaft | | |
| 23 | Längsachse Schaft | | |
| 24 | Maulbetätigungselement | | |
| 26 | Messerbetätigungselement | | |
| 28 | Wurfhebelsystem | | |
| 30 | Betätigungselement | | |
| 32 | Koppelglied | | |
| 34 | Wurfhebel | | |
| 36 | Gleitlager | | |
| 38 | Wurfhebel-Verbindungselement | | |
| 40 | Drehelement | | |
| 42 | Lastarm des Wurfhebels | | |
| 44 | Auflagepunkt Wurfhebel | | |
| 46 | Schwenkachse | | |

## Patentansprüche

1. Chirurgisches Maulinstrument (10), an dessen distalem Ende ein Maulteil (12) angeordnet ist, das mit zwei Maulbranchen (14, 16) ausgebildet ist, wobei mindestens eine Maulbranche schwenkbar gelagert ist und am proximalen Ende des Maulinstruments (10) ein Handgriff (18) zur Betätigung des Maulteils (12) angeordnet ist und das elektrochirurgische Maulinstrument eine reversibel in axialer Richtung verschiebbare Messeranordnung (20) zum Schneiden von Gewebe zwischen den Maulbranchen (14, 16) und einen als Rohr ausgebildeten Schaft (22) umfasst, an dessen distalem Ende das Maulteil (12) und an dessen proximalen Ende der Handgriff (18) angeordnet ist, wobei das Maulteil (12) von einem den Schaft (22) durchlaufenden Maulbetätigungselement (24) betätigbar ist und wobei die Messeranordnung (20) von einem den Schaft (22) durchlaufenden Messerbetätigungselement (26) betätigbar ist, und das Maulbetätigungselement (24) von dem am proximalen Ende des Schaftes (22) angeordneten Handgriff (18) in Richtung des Schaftes (22) längsverschiebbar ist, **dadurch gekennzeichnet, dass** das Messerbetätigungselement (26) mittels eines Wurfhebelsystems (28) in Richtung des Schaftes (22) längsverschiebbar ist, wobei das Wurfhebelsystem (28) ein Betätigungselement (30), ein Koppelglied (32) und einen Wurfhebel (34) umfasst und wobei das Messerbetätigungselement (26) durch Betätigung des Betätigungselements (30) innerhalb des Schaftes (22) in Schaftrichtung längsverschiebbar ist.

2. Chirurgisches Maulinstrument (10) nach Anspruch 1, **dadurch gekennzeichnet, dass** das Betätigungselement (30) mittels des Koppelglieds (32) mit dem Wurfhebel (34) funktional verbunden ist.

3. Chirurgisches Maulinstrument (10) nach einem der vorangegangenen Ansprüche, **dadurch gekennzeichnet, dass** das Betätigungselement (30) und das Koppelglied (32) mittels eines Lagers, das eine rotatorische Bewegung zulässt, verbunden sind oder dass das Betätigungselement (30) und das Koppelglied (32) durch eine starre Verbindung verbunden sind.

4. Chirurgisches Maulinstrument (10) nach einem der vorangegangenen Ansprüche, **dadurch gekennzeichnet, dass** der Wurfhebel (34) mit dem proximalen Endbereich des Messerbetätigungselements (26) verbunden ist.

5. Chirurgisches Maulinstrument (10) nach einem der vorangegangenen Ansprüche, **dadurch gekennzeichnet, dass** der Wurfhebel (34) mittels eines Gleitlagers (36) mit dem proximalen Endbereich des Messerbetätigungselements (26) verbunden ist.

6. Chirurgisches Maulinstrument (10) nach einem der vorangegangenen Ansprüche, **dadurch gekennzeichnet, dass** das Messerbetätigungselement (26) an seinem proximalen Ende ein Wurfhebel-Verbindungselement (38) aufweist, das einen Zapfenbereich (48) mit rundem Querschnitt und einen Verdickungsbereich (50) proximal des Zapfenbereichs (48) umfasst, und der Wurfhebel (34) an seinem mit dem Messerbetätigungselement (26) verbundenen Ende einen Schlitz (52) aufweist, wobei der Zapfenbereich (48) durch den Schlitz (52) derart hindurchgeführt ist, dass sich der Verdickungsbereich (50) im Inneren des Wurfhebels (34) befindet.

7. Chirurgisches Maulinstrument (10) nach einem der vorangegangenen Ansprüche, **dadurch gekennzeichnet, dass** das Messerbetätigungselement (26) mittels des Betätigungselements (30) um mindestens 30 mm in Längsrichtung des Schaftes (22) verschiebbar ist, vorzugsweise um mindestens 40 mm.

8. Chirurgisches Maulinstrument (10) nach einem der vorangegangenen Ansprüche, **dadurch gekennzeichnet, dass** das Maulteil (12) mittels eines im proximalen Endbereich des chirurgischen Maulelements angeordneten Drehelements (40) um die Längsachse (23) des Schaftes (22) drehbar ist, vorzugsweise in einem Winkelbereich zwischen 0° und 360°.

9. Chirurgisches Maulinstrument (10) nach einem der vorangegangenen Ansprüche, **dadurch gekennzeichnet, dass** das Drehelement (40) als Drehrad ausgebildet ist.

10. Chirurgisches Maulinstrument (10) nach einem der vorangegangenen Ansprüche, **dadurch gekennzeichnet, dass** das Drehelement (40) und das Betätigungselement (30) derart am oder in der Nähe des Handgriffs (18) angeordnet sind, dass eine einhändige Bedienung der Griffelemente des Handgriffs (18), des Drehelements (40) und des Betätigungselements (30) ermöglicht wird.

11. Chirurgisches Maulinstrument (10) nach einem der vorangegangenen Ansprüche, **dadurch gekennzeichnet, dass** der Lastarm (42) des Wurfhebels (34) um eine senkrecht durch seinen Auflagepunkt (44) und orthogonal zu der Längsachse (23) des Schafts (22) verlaufende Schwenkachse (46) von -45° bis + 45° schwenkbar ist.

## Claims

1. A surgical jaw-type instrument (10), on the distal end of which a jaw-type part (12) is arranged, which is formed with two jaw branches (14, 16), wherein at least one jaw branch is pivotably mounted, and a handle (18) for actuating the jaw-type part (12) is arranged on the proximal end of the jaw-type instrument (10), and the electrosurgical jaw-type instrument comprises a blade assembly (20), which can be moved reversibly in the axial direction, for cutting tissue between the jaw branches (14, 16), and a shaft (22) formed as tube, on the distal end of which the jaw-type part (12) is arranged and on the proximal end of which the handle (18) is arranged, wherein the jaw-type part (12) can be actuated by a jaw actuating element (24) passing through the shaft (22), and wherein the blade assembly (20) can be actuated by a blade actuating element (26) passing through the shaft (22), and the jaw actuating element (24) can be moved longitudinally in the direction of the shaft (22) by the handle (18) arranged on the proximal end of the shaft (22), **characterised in that** the blade actuating element (26) can be moved longitudinally in the direction of the shaft (22) by means of a counterweight lever system (28), wherein the counterweight lever system (28) comprises an actuating element (30), a coupling element (32), and a counterweight lever (34), and wherein the blade actuating element (26) can be moved longitudinally in the shaft direction by actuating the actuating element (30) within the shaft (22).

2. The surgical jaw-type instrument (10) according to claim 1, **characterised in that** the actuating element (30) is functionally connected to the counterweight lever (34) by means of the coupling element (32).

3. The surgical jaw-type instrument (10) according to any one of the preceding claims, **characterised in that** the actuating element (30) and the coupling element (32) are connected by means of a bearing, which permits a rotational movement, or that the actuating element (30) and the coupling element (32) are connected by means of a rigid connection.

4. The surgical jaw-type instrument (10) according to any one of the preceding claims, **characterised in that** the counterweight lever (34) is connected to the proximal end region of the blade actuating element (26).

5. The surgical jaw-type instrument (10) according to any one of the preceding claims, **characterised in that** the counterweight lever (34) is connected to the proximal end region of the blade actuating element (26) by means of a slide bearing (36).

6. The surgical jaw-type instrument (10) according to any one of the preceding claims, **characterised in that**, on its proximal end, the blade actuating element (26) has a counterweight lever connecting element (38), which comprises a journal region (48) comprising a round cross section, and a thickening region (50) proximal to the journal region (48), and, on its end connected to the blade actuating element (26), the counterweight lever (34) has a slot (52), wherein the journal region (48) is guided through the slot (52) in such a way that the thickening region (50) is located in the interior of the counterweight lever (34).

7. The surgical jaw-type instrument (10) according to any one of the preceding claims, **characterised in that** the blade actuating element (26) can be moved by at least 30 mm in the longitudinal direction of the shaft (22), preferably by at least 40 mm, by means of the actuating element (30).

8. The surgical jaw-type instrument (10) according to any one of the preceding claims, **characterised in that** the jaw-type part (12) can be rotated about the longitudinal axis (23) of the shaft (22), preferably in an angular range of between 0° and 360°, by means of a rotary element (40) arranged in the proximal end region of the surgical jaw-type element.

9. The surgical jaw-type instrument (10) according to any one of the preceding claims, **characterised in that** the rotary element (40) is formed as rotating wheel.

10. The surgical jaw-type instrument (10) according to any one of the preceding claims, **characterised in that** the rotary element (40) and the actuating element (30) are arranged on or in the vicinity of the handle (18) in such a way that a one-handed operation of the grip elements of the handle (18), of the rotary element (40), and of the actuating element (30) is made possible.

11. The surgical jaw-type instrument (10) according to any one of the preceding claims, **characterised in that** the load arm (42) of the counterweight lever (34) can be pivoted from -45° to +45° about a pivot axis (46), which runs perpendicular through its bearing point (44) and orthogonally to the longitudinal axis (23) of the shaft (22).

## Revendications

1. Instrument chirurgical (10) à mors à l'extrémité distale duquel est disposé une pièce dotée d'un mors (12) comportant deux branches (14, 16), l'une au moins des branches étant logée de façon à pouvoir pivoter, une poignée (18) permettant d'actionner la pièce à mors (12) étant disposée à l'extrémité proximale de l'instrument à mors (10), l'instrument à mors électrochirurgical comprenant un agencement de lames (20) déplaçable de façon réversible en direction axiale pour sectionner des tissus entre les branches du mors (14, 16) et une gaine (22) de conformation tubulaire à l'extrémité distale de laquelle est disposée la pièce à mors (12) et à l'extrémité proximale de laquelle est disposée la poignée (18), la pièce à mors (12) pouvant être actionnée par un élément actionneur de mors (24) qui traverse la gaine (22) et l'agencement de lames (20) pouvant être actionné par un élément actionneur de lames (26) qui traverse la gaine (22) et l'élément actionneur de mors (24) pouvant être déplacé longitudinalement en direction de la gaine (22) par la poignée (18) disposée à l'extrémité proximale de la gaine (22), **caractérisé en ce que** l'élément d'actionnement de lames (26) peut être déplacé longitudinalement dans le sens de la gaine (22) au moyen d'un système de levier monobras (28), ce système de levier monobras (28) comprenant un élément d'actionnement (30), une pièce de couplage (32) et un levier monobras (34) et l'élément d'actionnement de lames (26) pouvant être déplacé longitudinalement à l'intérieur de la gaine (22) dans le sens de la gaine.

2. Instrument chirurgical à mors (10) selon la revendication 1, **caractérisé en ce que** l'élément d'actionnement (30) est fonctionnellement relié au levier monobras (34) au moyen de la pièce de couplage (32)

3. Instrument chirurgical à mors (10) selon l'une des revendications précédentes, **caractérisé en ce que** l'élément d'actionnement (30) et la pièce de couplage (32) sont reliés au moyen d'un palier permettant un mouvement rotatoire, ou **en ce que** l'élément d'actionnement (30) et la pièce de couplage (32) sont reliés par un assemblage rigide.

4. Instrument chirurgical à mors (10) selon l'une des revendications précédentes, **caractérisé en ce que** le levier monobras (34) est relié à la section d'extrémité proximale de l'élément d'actionnement de lames (26).

5. Instrument chirurgical à mors (10) selon l'une des revendications précédentes, **caractérisé en ce que** le levier monobras (34) est relié à la section d'extrémité proximale de l'élément d'actionnement de lames (26) au moyen d'un palier lisse (36).

6. Instrument chirurgical à mors (10) selon l'une des revendications précédentes, **caractérisé en ce que** l'élément d'actionnement de lames (26) présente à son extrémité proximale un élément de liaison (38) pour le levier monobras, lequel élément comporte un corps (48) de section ronde et une partie plus épaisse (50) proximale par rapport au corps (48), et **en ce que** le levier monobras (34) présente à son extrémité reliée à l'élément d'actionnement de lames (26) une fente (52), le corps (48) étant introduit dans la fente (52) de façon à ce que la partie plus épaisse (50) soit logée à l'intérieur du levier monobras (34).

7. Instrument chirurgical à mors (10) selon l'une des revendications précédentes, **caractérisé en ce que** l'élément d'actionnement de lames (26) peut, au moyen de l'élément d'actionnement (30), être déplacé d'au moins 30 mm et de préférence d'au moins 40 mm dans le sens longitudinal de la gaine (22)

8. Instrument chirurgical à mors (10) selon l'une des revendications précédentes, **caractérisé en ce que** la pièce à mors (12) peut, au moyen d'un élément rotatif (40) placé dans la section d'extrémité proximale de l'instrument chirurgical à mors, pivoter d'un angle de préférence compris entre 0° et 360° autour de l'axe longitudinal (23) de la gaine (22).

9. Instrument chirurgical à mors (10) selon l'une des revendications précédentes, **caractérisé en ce que** l'élément rotatif (40) est réalisé sous forme de molette

10. Instrument chirurgical à mors (10) selon l'une des revendications précédentes, **caractérisé en ce que** l'élément rotatif (40) et l'élément d'actionnement (30) sont agencés au niveau ou à proximité de la poignée(18) de façon à ce que les éléments de manipulation de la poignée (18), de l'élément rotatif (40) et de l'élément d'actionnement (30) puissent être manipulés d'une seule main.

11. Instrument chirurgical à mors (10) selon l'une des revendications précédentes, **caractérisé en ce que** le bras de charge (42) du levier monobras (34) peut pivoter d'un angle de -45° à +45° autour d'un axe de pivotement (46) traversant verticalement son point d'appui (44) et orthogonal par rapport à l'axe longitudinal (23) de la gaine (22).
